# EUROPEAN PATENT APPLICATION

(11) **EP 4 620 465 A1**
(43) Date of publication of application: **24.09.2025**
(21) Application number: 23891593.8
(22) Date of filing: 15.11.2023
(51) Int. Cl.: A61K 31/202, A61K 31/232, A61K 31/683, A61K 31/7024, A61P 27/02, A61P 37/08

(54) **COMPOSITION FOR RELIEVING OR PREVENTING SYMPTOMS OF ALLERGIC RHINITIS OR ALLERGIC CONJUNCTIVITIS**

(30) Priority: 15.11.2022 JP 2022182658
(71) Applicant: Nissui Corporation, Tokyo 105-8676 (JP)
(72) Inventor: YOKOI, Kaori, Tokyo 105-8676 (JP); YANAGIMOTO, Kenichi, Tokyo 105-8676 (JP)
(74) Representative: Hoffmann Eitle
(86) International application number: PCT/JP2023/041009
(87) International publication number: WO 2024/106446

(57) **Abstract**

The present invention provides a composition for alleviating or preventing a symptom of allergic rhinitis or allergic conjunctivitis, the composition containing dihomo-γ-linolenic acid (DGLA) as an active ingredient.

## Description

### TECHNICAL FIELD

The present invention relates to a composition for alleviating or preventing a symptom of allergic rhinitis or allergic conjunctivitis.

### BACKGROUND ART

Dihomo-γ-linolenic acid (8,11, 14-eicosatrienoic acid; DGLA) is a polyunsaturated fatty acid of the n-6 series contained in breast milk, fish oil, seaweed, meat, and the like, and can be synthesized in a living body from linoleic acid via γ-linolenic acid. The DGLA is known to be present in biological membranes and blood like other fatty acids and to constitute 1 to 3% of biological membranes in the liver, spleen, epidermis, and the like.

It is known that γ-linolenic acid, the precursor of the DGLA, exhibits an antiinflammatory effect when orally administered. The effect is thought to be exhibited by the following mechanism: γ-linolenic acid is converted into the DGLA and further metabolized by cyclooxygenase to produce prostaglandin E1 (PGE1), which antagonizes prostaglandin E2, and that DGLA is metabolized by 15-lipoxygenase to produce 15-hydroxyeicosatrienoic acid (15-HETrE), which inhibits 5-lipoxygenase.

Regarding the DGLA, a production technique from microorganisms has been found (PTL 1).

Regarding the bioactivity of the DGLA, PTL 2 reports that the intake of DGLA suppresses dermatitis in dermatitis model animals. PTL 3 describes that, in an experiment using atopic dermatitis model mice, there was a tendency to suppress an increase in number of adipocytes in skin lesion sites by the intake of triglyceride containing DGLA as the main constituent fatty acid. PTL 4 does not illustrate an example using DGLA, but describes that oral administration of γ-linolenic acid (GLA) ethyl ester suppressed hyper IgE production in an experiment using atopic dermatitis model mice, and GLA suppressed IgE production in an in vitro experiment using splenocytes from the model mice. On the other hand, PTL 4 also describes that the suppression of IgE production was not observed even though GLA was orally administered for two months to atopic dermatitis-affected mice with hyper IgE-emia.

### CITATION LIST

### PATENT LITERATURE

PTL 1: Japanese Patent No. 3354581
PTL 2: WO2006/085687
PTL 3: Japanese Patent Laid-Open No. 2006-306813
PTL 4: Japanese Patent Laid-Open No. 2002-47176

### SUMMARY OF INVENTION

### TECHNICAL PROBLEM

There have been reports which suggest that the intake of DGLA suppresses atopic dermatitis in mice. However, there have been no reports which demonstrates the effect of the intake of DGLA on other allergic diseases.

PTL 2 and PTL 3 describe that, in the experiment using atopic dermatitis model mice, there was a tendency to suppress an increase in number of adipocytes in skin lesion sites by the intake of DGLA; however, it has been reported that the number of adipocytes does not necessarily increase in patients with Japanese cedar pollinosis (e.g., The Journal of the Oto-Rhino-Laryngological Society of Japan, Vol. 87, No. 2, 1984, pp.141-148). Therefore, there is no common technical knowledge that suppression of the increase in number of adipocytes can be enough to alleviate or prevent a symptom of allergic rhinitis or allergic conjunctivitis such as pollinosis.

The present invention aims to provide a novel means for alleviating or preventing a symptom of allergic rhinitis or allergic conjunctivitis.

### SOLUTION TO PROBLEM

The present inventors have found that the intake of DGLA reduces the severity of symptoms in subjects who have had nasal or eye discomfort, i.e., symptoms of allergic rhinitis or allergic conjunctivitis, for the past two years during the dispersal period of Japanese cedar or hinoki cypress pollen. Further, the present inventors have found that the intake of DGLA is more effective in reducing the severity of a symptom in subjects who have a symptom of allergic rhinitis or allergic conjunctivitis, in other words, subjects who are more sensitive to allergens, at the early stage of the dispersal period of pollen, which is an allergen.

The present inventors have conducted extensive studies based on these findings, and as a result, accomplished the present invention.

That is, the present invention is as follows.
[1] A composition for alleviating or preventing a symptom of allergic rhinitis or allergic conjunctivitis, comprising dihomo-γ-linolenic acid (DGLA) as an active ingredient.
[2] The composition according to [1], wherein the allergic rhinitis or allergic conjunctivitis is pollinosis.
[3] The composition according to [2], wherein the composition is used for oral administration to a subject having the symptom of the allergic rhinitis or allergic conjunctivitis at an early stage of a pollen dispersal period.
[4] The composition according to [3], wherein at least one score selected from sneezing attack, nasal discharge, nasal congestion, itchy eyes, and tearing in a severity classification of allergic rhinitis symptoms for the subject is 1 or higher at the early stage of the pollen dispersal period.
[5] The composition according to [3], wherein at least one score selected from runny nose, sneezing, blocked nose, itchy nose, itchy eyes, and watery eyes in the Japanese Allergic Rhinitis Standard Quality of Life Questionnaire for the subject is 1 or higher at the early stage of the pollen dispersal period.
[6] The composition according to [3], wherein a hinoki cypress pollen-specific IgE concentration in serum of the subject is 0.73 UA/mL or less.
[7] The composition according to any of [1] to [6], wherein the symptom is at least one selected from sneezing attack, nasal discharge, nasal congestion, itchy nose, itchy eyes, and tearing.
[8] The composition according to [7], wherein the symptom is at least one selected from sneezing attack, nasal discharge, itchy eyes, and tearing.
[9] The composition according to any of [1] to [8], wherein the DGLA of 4 mg/kg body weight/day or more is used for oral administration to the subject.
[10] The composition according to any of [1] to [9], wherein the DGLA is in any form of a glyceride, a phospholipid, a glycolipid, an alkyl ester, a salt, or a free fatty acid.
[11] The composition according to [10], wherein the glyceride is a triglyceride, a diglyceride, or a monoglyceride.
[12] The composition according to [10], wherein the alkyl ester is an ester with a lower alcohol having 1 to 4 carbon atoms.
[13] The composition according to [10], wherein the phospholipid is a diacylglycerophospholipid or a lysoacylglycerophospholipid.
[14] A method for alleviating or preventing a symptom of allergic rhinitis or allergic conjunctivitis, comprising administering to a subject an effective amount of a composition containing dihomo-γ-linolenic acid (DGLA) as an active ingredient.
[15] The method according to [14], wherein the allergic rhinitis or allergic conjunctivitis is pollinosis.
[16] The method according to [15], wherein the subject has the symptom of the allergic rhinitis or allergic conjunctivitis at an early stage of a pollen dispersal period.
[17] The method according to [16], wherein at least one score selected from sneezing attack, nasal discharge, nasal congestion, itchy eyes, and tearing in a severity classification of allergic rhinitis symptoms for the subject is 1 or higher at the early stage of the pollen dispersal period.
[18] The method according to [15], wherein at least one score selected from runny nose, sneezing, blocked nose, itchy nose, itchy eyes, and watery eyes in the Japanese Allergic Rhinitis Standard Quality of Life Questionnaire for the subject is 1 or higher at the early stage of the pollen dispersal period.
[19] The method according to [16], wherein a hinoki cypress pollen-specific IgE concentration in serum of the subject is 0.73 UA/mL or less.
[20] The method according to any of [14] to [19], wherein the symptom is at least one selected from sneezing attack, nasal discharge, nasal congestion, itchy nose, itchy eyes, and tearing.
[21] The method according to [20], wherein the symptom is at least one selected from sneezing attack, nasal discharge, itchy eyes, and tearing.
[22] The method according to any of [14] to [21], wherein the DGLA of 4 mg/kg body weight/day or more is administered to the subject.
[23] The method according to any of [14] to [22], wherein the DGLA is in any form of a glyceride, a phospholipid, a glycolipid, an alkyl ester, a salt, or a free fatty acid.
[24] The method according to [23], wherein the glyceride is a triglyceride, a diglyceride, or a monoglyceride.
[25] The method according to [23], wherein the alkyl ester is an ester with a lower alcohol having 1 to 4 carbon atoms.
[26] The method according to [23], wherein the phospholipid is a diacylglycerophospholipid or a lysoacylglycerophospholipid.
[27] A composition for use in alleviating or preventing a symptom of allergic rhinitis or allergic conjunctivitis, comprising dihomo-γ-linolenic acid (DGLA) as an active ingredient.
[28] The composition according to [27], wherein the allergic rhinitis or allergic conjunctivitis is pollinosis.
[29] The composition according to [28], wherein the composition is used for oral administration to a subject having the symptom of the allergic rhinitis or allergic conjunctivitis at an early stage of a pollen dispersal period.
[30] The composition according to [29], wherein at least one score selected from sneezing attack, nasal discharge, nasal congestion, itchy eyes, and tearing in a severity classification of allergic rhinitis symptoms for the subject is 1 or higher at the early stage of the pollen dispersal period.
[31] The composition according to [29], wherein at least one score selected from runny nose, sneezing, blocked nose, itchy nose, itchy eyes, and watery eyes in the Japanese Allergic Rhinitis Standard Quality of Life Questionnaire for the subject is 1 or higher at the early stage of the pollen dispersal period.
[32] The composition according to [29], wherein a hinoki cypress pollen-specific IgE concentration in serum of the subject is 0.73 UA/mL or less.
[33] The composition according to any of [27] to [32], wherein the symptom is at least one selected from sneezing attack, nasal discharge, nasal congestion, itchy nose, itchy eyes, and tearing.
[34] The composition according to [33], wherein the symptom is at least one selected from sneezing attack, nasal discharge, itchy eyes, and tearing.
[35] The composition according to any of [27] to [34], wherein the DGLA of 4 mg/kg body weight/day or more is used for oral administration to the subject.
[36] The composition according to any of [27] to [35], wherein the DGLA is in any form of a glyceride, a phospholipid, a glycolipid, an alkyl ester, a salt, or a free fatty acid.
[37] The composition according to [36], wherein the glyceride is a triglyceride, a diglyceride, or a monoglyceride.
[38] The composition according to [36], wherein the alkyl ester is an ester with a lower alcohol having 1 to 4 carbon atoms.
[39] The composition according to [36], wherein the phospholipid is a diacylglycerophospholipid or a lysoacylglycerophospholipid.
[40] The composition according to [27] to [39], wherein the composition is a pharmaceutical composition, a food composition, or a food additive.
[41] The composition according to [27] to [40], wherein the DGLA of 4 mg/kg body weight/day to 500 mg/kg body weight/day is used for oral administration to the subject.
[42] The composition according to [27] to [41], wherein the composition is orally administered for a week or longer.
[43] The composition according to [28], wherein an intake of the composition is started before a first day of pollen dispersal or at an early stage of a pollen dispersal period.
[44] Use of a composition comprising dihomo-γ-linolenic acid (DGLA) as an active ingredient, for producing a composition of alleviating or preventing a symptom of allergic rhinitis or allergic conjunctivitis.
[45] The use according to [44], wherein the allergic rhinitis or allergic conjunctivitis is pollinosis.
[46] The use according to [45], wherein the composition is used for oral administration to a subject having the symptom of the allergic rhinitis or allergic conjunctivitis at an early stage of a pollen dispersal period.
[47] The use according to [46], wherein at least one score selected from sneezing attack, nasal discharge, nasal congestion, itchy eyes, and tearing in a severity classification of allergic rhinitis symptoms for the subject is 1 or higher at the early stage of the pollen dispersal period.
[48] The use according to [46], wherein at least one score selected from runny nose, sneezing, blocked nose, itchy nose, itchy eyes, and watery eyes in the Japanese Allergic Rhinitis Standard Quality of Life Questionnaire for the subject is 1 or higher at the early stage of the pollen dispersal period.
[49] The use according to [46], wherein a hinoki cypress pollen-specific IgE concentration in serum of the subject is 0.73 UA/mL or less.
[50] The use according to any of [44] to [49], wherein the symptom is at least one selected from sneezing attack, nasal discharge, nasal congestion, itchy nose, itchy eyes, and tearing.
[51] The use according to [50], wherein the symptom is at least one selected from sneezing attack, nasal discharge, itchy eyes, and tearing.
[52] The use according to any of [44] to [51], wherein the DGLA of 4 mg/kg body weight/day or more is used for oral administration to the subject.
[53] The use according to any of [44] to [52], wherein the DGLA is in any form of a glyceride, a phospholipid, a glycolipid, an alkyl ester, a salt, or a free fatty acid.
[54] The use according to [53], wherein the glyceride is a triglyceride, a diglyceride, or a monoglyceride.
[55] The use according to [53], wherein the alkyl ester is an ester with a lower alcohol having 1 to 4 carbon atoms.
[56] The use according to [53], wherein the phospholipid is a diacylglycerophospholipid or a lysoacylglycerophospholipid.

### ADVANTAGEOUS EFFECTS OF INVENTION

The intake of the composition of the present invention enables alleviation or prevention of a symptom of allergic rhinitis or allergic conjunctivitis.

### BRIEF DESCRIPTION OF DRAWINGS

[Fig. 1] Fig. 1 shows an increase or decrease of sneezing attack score in the severity classification of allergic rhinitis symptoms from the start of testing (week 0). p < 0.01.
[Fig. 2] Fig. 2 shows an increase or decrease of nasal congestion score in the severity classification of allergic rhinitis symptoms from the start of testing (week 0). p < 0.05.
[Fig. 3] Fig. 3 shows an increase or decrease of nasal/eye symptom score in the Japanese Allergic Rhinitis Standard Quality of Life Questionnaire (JRQLQ) from the start of testing (week 0). p < 0.1.
[Fig. 4] Fig. 4 shows results of stratification analysis of the DGLA group on the sneezing attack score in the severity classification of allergic rhinitis symptoms. Group H: n=15; Group L: n=3. P=0.712.
[Fig. 5] Fig. 5 shows results of stratification analysis of the DGLA group on the nasal discharge score in the severity classification of allergic rhinitis symptoms. Group H: n=15; Group L: n=3. P=0.258.
[Fig. 6] Fig. 6 shows results of stratification analysis of the DGLA group on the nasal congestion score in the severity classification of allergic rhinitis symptoms. Group H: n=8; Group L: n=10. P=0.249.
[Fig. 7] Fig. 7 shows results of stratification analysis of the DGLA group on the itchy eyes score in the severity classification of allergic rhinitis symptoms. Group H: n=14; Group L: n=4. P=0.303.
[Fig. 8] Fig. 8 shows results of stratification analysis of the DGLA group on the tearing score in the severity classification of allergic rhinitis symptoms. Group H: n=7; Group L: n=11. P=0.284.
[Fig. 9] Fig. 9 shows results of stratification analysis of the DGLA group on the runny nose score in JRQLQ. Group H: n=15; Group L: n=3. P=0.582.
[Fig. 10] Fig. 10 shows results of stratification analysis of the DGLA group on the sneezing score in JRQLQ. Group H: n=16; Group L: n=2. P=0.345.
[Fig. 11] Fig. 11 shows results of stratification analysis of the DGLA group on the blocked nose score in JRQLQ. Group H: n=13; Group L: n=5. P=0.126.
[Fig. 12] Fig. 12 shows results of stratification analysis of the DGLA group on the itchy nose score in JRQLQ. Group H: n=10; Group L: n=8. P=0.084.
[Fig. 13] Fig. 13 shows results of stratification analysis of the DGLA group on the itchy eyes score in JRQLQ. Group H: n=12; Group L: n=6. P=0.362.
[Fig. 14] Fig. 14 shows results of stratification analysis of the DGLA group on the watery eyes score in JRQLQ. Group H: n=12; Group L: n=6. P=0.59.

### DESCRIPTION OF EMBODIMENTS

The present invention provides a composition for alleviating or preventing a symptom of allergic rhinitis or allergic conjunctivitis, the composition comprising DGLA as an active ingredient (hereinafter, also referred to as the composition of the present invention).

In the present invention, dihomo-γ-linolenic acid (DGLA) is an unsaturated fatty acid of the n-6 series having 20 carbon atoms and 3 double bonds. The DGLA used for the purpose of the present invention may be in any form of a free fatty acid, a salt such as sodium or potassium, a lower alkyl ester having 1 to 4 carbon atoms such as an ethyl ester or a methyl ester, a glyceride (glycerin ester) such as a triglyceride, a diglyceride, or a monoglyceride, a phospholipid such as a diacylglycerophospholipid, or a lysoacylglycerophospholipid, or a glycolipid, as long as the DGLA is contained as a constituent fatty acid. The DGLA may be preferably in the form of an ester such as a glyceride or a lower alkyl ester, and more preferably in the form of a glyceride, and even more preferably in the form of a triglyceride.

The DGLA can be mass-produced by the method disclosed in Japanese Patent No. 3354581. Specifically, microorganisms of the genus Mortierella, in particular, Mortierella alpina, that produce DGLA-containing fat or oil are cultured to obtain DGLA by extracting fat or oil from fungal cells. By this method, fat or oil in which the content (ratio) of the DGLA in the fatty acid composition is 30 to 50% by weight can be obtained. In the present invention, the fat or oil may be used as it is, or the DGLA may further be concentrated for use. Specifically, fat and oil containing DGLA can be concentrated by a wintering treatment, an enzymatic treatment with lipase, or the like. Alternatively, fat or oil containing DGLA can be converted by hydrolysis or alcoholysis into a free fatty acid, a salt thereof, an ester with a lower alcohol, or the like for use. Further, it can be concentrated by performing distillation treatment, urea addition treatment, column treatment, enzymatic treatment, supercritical carbon dioxide treatment, or the like.

The concentration of DGLA in the composition of the present invention, in other words, the ratio of DGLA to the total fatty acid in the fatty acid composition of the composition of the present invention, may be about 0.01 to 50% by weight, for example, 0.05 to 50% by weight, 0.1 to 50% by weight, 0.5 to 50% by weight, 1.0 to 50% by weight, 5.0 to 50% by weight, 10 to 50% by weight, 20 to 50% by weight, 30 to 50% by weight, 30 to 45% by weight, 30 to 40% by weight, or 30 to 35% by weight, and is preferably 30 to 35% by weight.

As used herein, the ratio (% by weight) of fatty acid to the total fatty acid in the fatty acid composition of the composition is calculated based on values measured by gas chromatography after esterification of the components in the composition according to "AOCS official method Ce1B-89", unless otherwise explicitly stated. When also referred to as the concentration or content of fatty acid, it means the ratio (% by weight) of the fatty acid to the total fatty acid described above. The analysis conditions for gas chromatography are as follows.
Apparatus: Agilent6850 GC system (Agilent, Inc.)
Column: DB-WAX (Agilent Technologies, Inc., 30 m × 0.32 mm ID, 0.25 µm film thickness)
Carrier gas: Helium (2.2 mL/min, constant flow)
Infusion port temperature: 250°C
Amount to be infused: 1 µL
Infusion method: Split
Split ratio: 50:1
Column oven: 170°C - 1°C/min - 225°C
Detector: FID
Detector temperature: 300°C

The term "allergic rhinitis" in the present invention refers to a disease in which an immunoreaction occurs when an allergen enters the body, causing nasal symptoms such as sneezing attacks, nasal discharge, and nasal congestion. The term "allergic conjunctivitis" in the present invention refers to a disease in which an immunoreaction occurs when an allergen enters the body, causing eye symptoms such as itchy eyes and tearing. The allergic rhinitis or allergic conjunctivitis includes perennial and seasonal ones. The perennial allergic rhinitis or allergic conjunctivitis can occur at any time of the year, due to house dust (e.g., fungal spores, textile fibers, animal dander, mites, and insect cadavers), or the like. The seasonal allergic rhinitis or allergic conjunctivitis can occur during a specific period of the year due to pollen or the like.

In a preferred embodiment of the present invention, the allergic rhinitis or allergic conjunctivitis can be seasonal allergic rhinitis or allergic conjunctivitis, and more preferably pollinosis. Examples of pollinosis include Japanese cedar pollinosis, hinoki cypress pollinosis, ragweed pollinosis, rice pollen allergy, zelkova pollen allergy, orchard grass pollen allergy, white birch pollen allergy, Quercus serrata pollen allergy, alder pollen allergy, and Pinus pollen allergy. In a preferred embodiment of the present invention, the allergic rhinitis or allergic conjunctivitis can be Japanese cedar or hinoki cypress pollinosis.

The term "having the symptom of the allergic rhinitis or allergic conjunctivitis" in the present invention refers to satisfying at least one of the following (i) and (ii).
(i) At least one score selected from sneezing attack, nasal discharge, nasal congestion, itchy eyes, and tearing in the severity classification of allergic rhinitis symptoms is 1 or higher.
(ii) At least one score selected from runny nose, sneezing, blocked nose, itchy nose, itchy eyes, and watery eyes in the Japanese Allergic Rhinitis Standard Quality of Life Questionnaire (JRQLQ) for the subject is 1 or higher.

Therefore, the subject in the present invention may not have been diagnosed by a physician as suffering from "allergic rhinitis or allergic conjunctivitis" as long as the subject has subjective symptoms indicated by satisfying at least one of the above (i) and (ii). In one embodiment, the subject in the present invention may be a person who has nasal or eye discomfort due to an allergen such as pollen, but does not suffer from "allergic rhinitis or allergic conjunctivitis" (or at least a person who has not been diagnosed by a physician as suffering from "allergic rhinitis or allergic conjunctivitis").

The severity classification of allergic rhinitis symptoms refers to a method of classifying the severity based on Practical Guideline for the Management of Allergic Rhinitis in Japan 2020 (Committee of the Practical Guideline for the Management of Allergic Rhinitis). Specifically, each evening, the subject himself/herself selects the one that best describes the severity of the day on the subjective symptoms on each item listed in the table below.

**[Table 1]**

| | **0** | **1** | **2** | **3** | **4** |
|---|---|---|---|---|---|
| Sneezing attacks (the average number of attacks per day) | 0 times | 1 to 5 times | 6 to 10 times | 11 to 20 times | 21 times or more |
| Nasal discharge (the average number of times nasal blowing occurs per day) | 0 times | 1 to 5 times | 6 to 10 times | 11 to 20 times | 21 times or more |
| Nasal congestion | No nasal congestion | Nasal congestion without oral breathing | Severe nasal congestion with occasional oral breathing in a day | Very severe nasal congestion with oral breathing for a significant amount of time in a day | Completely blocked all day |
| Itchy eyes | None | Not severe for causing eye rubbing | Occasional eye rubbing | Frequent eye rubbing | Equal degree to or more than 3 |
| Tearing | None | Not severe for tear wiping | Occasional tear wiping | Frequent tear wiping | Equal degree to or more than 3 |
| Degree of interference in daily life | None | Minimal | Degree between 1 and 3 | Hard to do anything | Unable to do anything |

The JRQLQ is a questionnaire created by Okuda et al. to assess the impact of allergic rhinitis on quality of life (QOL) (Minoru Okuda et al.: Japanese Rhino-conjunctivitis Quality-of-Life Questionnaire (2002 edition), Committee of the Japanese Rhino-conjunctivitis Quality-of-Life Questionnaire, Allergy 2003; 52: 21-56). The JRQLQ has been reported to be significantly correlated with the Japanese version of the Rhino-conjunctivitis Quality of Life Questionnaire; RQLQJ, which is the Japanese translation of the Rhino-conjunctivitis Quality of Life Questionnaire (RQLQ) used worldwide (Japanese Journal of Rhinology, 52(4): 499-505, 2013). In the JRQLQ, the subject himself/herself selects the one that best describes the severity of the worst nasal and eye symptoms the subject has experienced in the past 1-2 weeks on the items listed in the table below.

**[Table 2]**

| | **0** | **1** | **2** | **3** | **4** |
|---|---|---|---|---|---|
| Runny nose | No symptoms | Mild | Moderate | Severe | Very severe |
| Sneezing | No symptoms | Mild | Moderate | Severe | Very severe |
| Blocked nose | No symptoms | Mild | Moderate | Severe | Very severe |
| Itchy nose | No symptoms | Mild | Moderate | Severe | Very severe |
| Itchy eyes | No symptoms | Mild | Moderate | Severe | Very severe |
| Watery eyes | No symptoms | Mild | Moderate | Severe | Very severe |

In the present invention, the symptom of the allergic rhinitis or allergic conjunctivitis is not particularly limited, and may be at least one selected from sneezing attack, nasal discharge, nasal congestion, itchy nose, itchy eyes, and tearing.

As shown in Examples to be described later, the composition of the present invention can significantly reduce the score of sneezing attack or nasal congestion in the severity classification of allergic rhinitis symptoms, in particular, or suppress the increase of the score, compared to a placebo group. Accordingly, in a preferred embodiment of the present invention, the symptom of the allergic rhinitis or allergic conjunctivitis may be at least one selected from sneezing and nasal congestion. In addition, the composition of the present invention can be used for reducing the score of sneezing attack or nasal congestion in the severity classification of allergic rhinitis symptoms, or suppressing the increase of the score, compared to the placebo group.

Also, as shown in Examples to be described later, the composition of the present invention can reduce the scores of nasal and eye symptoms in the JRQLQ, in particular, or suppress the increase of the score, compared to the placebo group. In the present invention, the phrase the "score of nasal and eye symptoms in the JRQLQ" means the sum of the scores of runny nose, sneezing, blocked nose, itchy nose, itchy eyes, and watery eyes in the JRQLQ. In a preferred embodiment of the present invention, the composition of the present invention can be used for reducing the scores of nasal and eye symptoms in the JRQLQ, or suppressing the increase of the score, compared to the placebo group.

Among the symptoms of the allergic rhinitis or allergic conjunctivitis, sneezing attack, nasal discharge, itchy eyes, and tearing are known to be due to the effects of histamine. It has not been reported that the intake of the DGLA affects the effects of histamine in the allergic rhinitis or allergic conjunctivitis. In a preferred embodiment of the present invention, the symptom of the allergic rhinitis or allergic conjunctivitis may be at least one selected from symptoms mediated by histamine, namely, sneezing attack, nasal discharge, itchy eyes, and tearing.

A subject to whom the composition of the present invention is administered is a mammal, and preferably a human. In one embodiment, the composition of the present invention can be administered to a subject in need of alleviating or preventing the symptom of the allergic rhinitis or allergic conjunctivitis. The composition of the present invention can also be used for non-therapeutic application.

In one embodiment, when the allergic rhinitis or allergic conjunctivitis is pollinosis, the composition of the present invention can be used for administration to a subject having the symptom of the allergic rhinitis or allergic conjunctivitis at the early stage of the dispersal period of pollen, which is an allergen (e.g., Japanese cedar or hinoki cypress pollen). The phrase the "early stage of the pollen dispersal period" as used in the present invention refers to a period before the first day of pollen dispersal but when a trace of pollen dispersal is detected in the region where the subject resides (e.g., prefectures or municipalities in Japan). In one embodiment, the phrase the "early stage of the pollen dispersal period" can be a period from 6 weeks before the first day of pollen dispersal to the first day of pollen dispersal in the region where the subject resides (e.g., prefectures or municipalities in Japan). Here, the phrase the "first day of pollen dispersal" refers to the first day on which one or more pollen grains per 1 cm² measured by Durham's method are observed in 2 consecutive days immediately before (e.g., 2 weeks to 1 day before) the period when the amount of dispersal of pollen, which is an allergen, begins to increase, in the region where the subject resides (e.g., prefectures or municipalities in Japan). In particular, the first day of Japanese cedar pollen dispersal in Japan is defined according to the definition by Japan Allergy Foundation, public interest incorporated foundation, Committee for Standardization of Pollen Information.

The "early stage of the pollen dispersal period" can be, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, and from 6 weeks to 5 weeks before the first day of pollen dispersal, and preferably can be a period from 6 weeks to 5 weeks before the first day of pollen dispersal. At the early stage of the pollen dispersal period, a trace of pollen is dispersed, and the subject having symptom of the allergic rhinitis or allergic conjunctivitis at the early stage of the pollen dispersal period can be said to be a subject highly sensitive to pollen, which is the allergen causing the symptoms. The composition of the present invention can exhibit higher effects on such a subject.

In one embodiment, the subject having the symptom of the allergic rhinitis or allergic conjunctivitis at the early stage of the pollen dispersal period can be, for example, a subject who has at least one score of 1 or higher selected from sneezing attack, nasal discharge, nasal congestion, itchy eyes, and tearing in the severity classification of allergic rhinitis symptoms at the early stage of the pollen dispersal period, or a subject who has at least one score of 1 or higher selected from runny nose, sneezing, blocked nose, itchy nose, itchy eyes, and watery eyes in the Japanese Allergic Rhinitis Standard Quality of Life Questionnaire at the early stage of the pollen dispersal period.

In one embodiment, the subject having the symptom of the allergic rhinitis or allergic conjunctivitis at the early stage of the pollen dispersal period, and preferably the subject having the itchy nose score of 1 or higher in the Japanese Allergic Rhinitis Standard Quality of Life Questionnaire at the early stage of the pollen dispersal period can have a hinoki cypress pollen-specific IgE concentration in serum of 0.73 UA/mL or less.

The composition of the present invention can be prepared in the form suitable for a pharmaceutical composition, a food composition (e.g., functional food, health food, and supplements), and a food additive, for example, various solid preparations such as granules (including dry syrup), capsules (soft capsules and hard capsules), tablets (including chewable tablets), powdered drugs (powders), and pills, or liquid preparations such as oral administration liquids (including liquids, suspensions, and syrup). The composition of the present invention can be, for example, formulated as a soft capsule in which the active ingredient is filled into a gelatin membrane.

Examples of the additive for formulation include an excipient, a lubricant, a binder, a disintegrant, a fluidizing agent, a dispersant, a humectant, a preservative, a thickening agent, a pH adjuster, a colorant, a corrigent, a surfactant, and a solubilizer. In addition, when making it in the form of a liquid, thickeners such as pectin, xanthan gum, and guar gum can be blended. Also, a coated tablet can be made by using a coating agent, and a paste-like glue can also be made. Further, preparation in other forms can also be made according to conventional methods.

The composition of the present invention can be in the form of a food composition or a food additive. In the present invention, the food composition means food in general including beverages, and examples thereof include general food including health food such as supplements, and food for specified health uses and food with nutrient function claims regulated by the System of Foods with Health Claims managed by the Consumer Affairs Agency. For example, provided are functional foods with a label indicating that nasal and/or eye discomfort due to an allergen such as pollen can be alleviated. For example, fat and oil containing the DGLA can be provided as it is. The food composition or food additive of the present invention also includes food materials that are added to, mixed with, or applied to other foods to impart effects of alleviating or preventing a symptom of the allergic rhinitis or allergic conjunctivitis. In addition to food, The food composition or food additive of the present invention can also be provided as animal feed.

The food composition or food additive of the present invention can be prepared by blending the composition of the present invention into a food composition or food additive, and thereby imparting a function of alleviating or preventing a symptom of the allergic rhinitis or allergic conjunctivitis.

The food composition of the present invention may be beverages, confectionery, bread, soup, and the like, and examples thereof include common retort foods, frozen foods, instant foods (e.g., noodles), canned foods, sausages, cookies, biscuits, cereal bars, crackers, snacks (e.g., potato chips), pastries, cake, pies, candies, chewing gum (including pellets and sticks), jelly, soup, ice cream, dressing, yogurt, supplements in the form of tablets, capsules, emulsions, and the like, and soft drinks. The method for producing these food products is not particularly limited as long as the effects of the present invention are not impaired, and may be performed for each food product according to methods used by those skilled in the art.

It is within the scope of the present invention to sell a product according to the present invention by displaying the effects of the composition of the present invention on packaging containers, product instructions, and pamphlets. It is also within the scope of the present invention to advertise and sell a product according to the present invention by displaying the effects of the present invention on television, Internet websites, pamphlets, newspapers, magazines, and the like. When the composition of the present invention is made into a medicine or a quasi-drug, its efficacy and/or effects of alleviating or preventing the symptom of the allergic rhinitis or allergic conjunctivitis can be displayed on the packaging container, or the like.

In the present invention, the amount of active ingredients to be taken by the subject is not particularly limited, and for example, an amount equal to or more than the effective amount in order to obtain a desired effect is taken. Here, the effective amount in order to obtain the desired effect refers to an amount necessary to alleviate or prevent the symptom of the allergic rhinitis or allergic conjunctivitis. For example, in a case of an adult, the DGLA (in terms of free fatty acid) as the active ingredient can be taken in an amount of 4 mg/kg body weight/day or more, for example, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more, depending on conditions such as the age, body weight, health condition of the subject. In addition, since the active ingredient of the composition of the present invention does not have a strong side effect, there is no upper limit to the daily amount of intake, and for example, the amount can be 500 mg/kg body weight/day or less, 400 mg/kg body weight/day or less, 200 mg/kg body weight/day or less, or 150 mg/kg body weight/day or less. In one embodiment, the composition of the present invention can be used such that the DGLA is orally administered to the subject in an amount of 4 mg/kg body weight/day to 500 mg/kg body weight/day, for example, 4 mg/kg body weight/day to 400 mg/kg body weight/day, 4 mg/kg body weight/day to 200 mg/kg body weight/day, 4 mg/kg body weight/day to 150 mg/kg body weight/day, 4 mg/kg body weight/day to 50 mg/kg body weight/day, 4 mg/kg body weight/day to 10 mg/kg body weight/day, 4 mg/kg body weight/day to 9 mg/kg body weight/day, 4 mg/kg body weight/day to 8 mg/kg body weight/day, or 4 mg/kg body weight/day to 7 mg/kg body weight/day.

In one embodiment, the composition of the present invention can be used such that the DGLA (in terms of free fatty acid) as the active ingredient is orally administered to the subject in an amount of 314 mg/day or more, for example, 400 mg/day or more, 500 mg/day or more, 600 mg/day or more, 700 mg/day or more, 800 mg/day or more, 900 mg/day or more, or 1000 mg/day or more.

The intake duration of the composition of the present invention is not particularly limited, and examples thereof may be 1 week or more, for example, 2 weeks or more, 3 weeks or more, 4 weeks or more, 5 weeks or more, 6 weeks or more, 7 weeks or more, 8 weeks or more, 9 weeks or more, 10 weeks or more, 11 weeks or more, 12 weeks or more, 13 weeks or more, 14 weeks or more, or 15 weeks or more.

In one embodiment, when the allergic rhinitis or allergic conjunctivitis is pollinosis, the composition of the present invention can be used such that the intake is started before the first day of pollen dispersal, for example, 1 week before or earlier, 2 weeks before or earlier, 3 weeks before or earlier, 4 weeks before or earlier, 5 weeks before or earlier, or 6 weeks before or earlier the first day of pollen dispersal. In another embodiment, the composition of the present invention can be used such that the intake is started at the early stage of the pollen dispersal period, for example, 6 weeks to 1 week before the first day of pollen dispersal, 6 weeks to 2 weeks before the first day of pollen dispersal, 6 weeks to 3 weeks before the first day of pollen dispersal, 6 weeks to 4 weeks before the first day of pollen dispersal, or 6 weeks to 5 weeks before the first day of pollen dispersal. In addition, the composition of the present invention can be taken until the last day of pollen dispersal, or may be continuously taken even after the last day of pollen dispersal. The phrase the "last day of pollen dispersal" in the present invention refers to the day before the first day on which 0 pollen grains per 1 cm² measured by Durham's method are observed in 3 consecutive days after the peak of the amount of dispersal in the region where the subject resides (e.g., prefectures or municipalities in Japan).

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is sneezing attack,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is nasal congestion,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is sneezing attack,
the composition is used for oral administration to the subject who has the sneezing attack score of 1 or higher in the severity classification of allergic rhinitis symptoms at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is sneezing attack,
the composition is used for oral administration to the subject who has the sneezing attack score of 1 or higher in the severity classification of allergic rhinitis symptoms at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is nasal discharge,
the composition is used for oral administration to the subject who has the nasal discharge score of 1 or higher in the severity classification of allergic rhinitis symptoms at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is nasal congestion,
the composition is used for oral administration to the subject who has the nasal congestion score of 1 or higher in the severity classification of allergic rhinitis symptoms at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is itchy eyes,
the composition is used for oral administration to the subject who has the itchy eyes score of 1 or higher in the severity classification of allergic rhinitis symptoms at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is tearing,
the composition is used for oral administration to the subject who has the tearing score of 1 or higher in the severity classification of allergic rhinitis symptoms at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is nasal discharge,
the composition is used for oral administration to the subject who has the runny nose score of 1 or higher in the JRQLQ at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is sneezing attack,
the composition is used for oral administration to the subject who has the sneezing score of 1 or higher in the JRQLQ at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is nasal congestion,
the composition is used for oral administration to the subject who has the blocked nose score of 1 or higher in the JRQLQ at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is itchy nose,
the composition is used for oral administration to the subject who has the itchy nose score of 1 or higher in the JRQLQ at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is itchy eyes,
the composition is used for oral administration to the subject who has the itchy eyes score of 1 or higher in the JRQLQ at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In a preferred embodiment, the composition of the present invention may be a composition for alleviating or preventing a symptom of allergic rhinitis or allergic conjunctivitis, the composition comprising the DGLA as an active ingredient, wherein the allergic rhinitis or allergic conjunctivitis is pollinosis, preferably Japanese cedar or hinoki cypress pollinosis,
the symptom of the allergic rhinitis or allergic conjunctivitis is tearing,
the composition is used for oral administration to the subject who has the watery eyes score of 1 or higher in the JRQLQ at the early stage of the pollen dispersal period, for example, a period from 6 weeks to 1 week before the first day of pollen dispersal, from 6 weeks to 2 weeks before the first day of pollen dispersal, from 6 weeks to 3 weeks before the first day of pollen dispersal, from 6 weeks to 4 weeks before the first day of pollen dispersal, or from 6 weeks to 5 weeks before the first day of pollen dispersal, and preferably a period from 6 weeks to 5 weeks before the first day of pollen dispersal,
the DGLA is a triglyceride, preferably a triglyceride, and
the composition is used such that the DGLA is orally administered to the subject in an amount of 4 mg/kg body weight/day or more, 5 mg/kg body weight/day or more, 6 mg/kg body weight/day or more, 7 mg/kg body weight/day or more, 8 mg/kg body weight/day or more, 9 mg/kg body weight/day or more, 10 mg/kg body weight/day or more, 50 mg/kg body weight/day or more, or 100 mg/kg body weight/day or more.

In an aspect, the present invention can provide a method for alleviating or preventing a symptom of allergic rhinitis or allergic conjunctivitis, comprising administering to a subject an effective amount of the composition of the present invention. The method can be performed according to the description herein regarding the intake or administration of the composition of the present invention.

### EXAMPLES

The present invention will be further described referring to Examples described below; however, the present invention is not limited to these.

### Example 1

### 1. Materials and Methods

### (1) Subjects

The subjects were adult males and females who had experienced nasal and/or eye discomfort due to pollen for the past 2 years. An eligibility assessment was conducted on 49 subjects, and 13 people who did not meet the following criteria were excluded.
· A healthy Japanese male or female at the age of 20 or older
· A person who has had nasal and/or eye discomfort due to pollen for the past 2 years.
· A person who does not use any therapeutic agent for allergy (excluding those who sometimes use a therapeutic agent for allergy for nasal and/or eye discomfort due to pollen)

The remaining 36 subjects were randomized and divided into a DGLA administration group (intervention group; hereinafter referred to as DGLA group) with 18 subjects and a placebo administration group (control group; hereinafter referred to as placebo group) with 18 subjects. 3 subjects in the placebo group became untraceable during the testing. Therefore, the analysis was performed on 18 subjects in the DGLA group and 15 subjects in the placebo group.

Data for the analyzed subjects is shown below.

**[Table 3]**

| | **DGLA group (n=18)** | **Placebo group (n=15)** |
|---|---|---|
| n (male/female) | 7/11 | 4/11 |
| Age (yr) | 48.1±11.6 | 49.7±13.0 |
| Body weight (kg) | 59.3±14.5 | 61.3±24.5 |
| BMI (kg/m²) | 21.9±3.4 | 23.4±7.2 |
| Japanese cedar-specific IgE concentration (UA/mL) | 10.8±15.9 | 14.3±19.5 |
| Hinoki cypress-specific IgE concentration (UA/mL) | 1.3±1.6 | 2.8±3.2 |

### (2) Diet for Testing

The DGLA group: DGLA-containing oil (DGLA of 31.4% by weight) was taken at 1,000 mg/day for 15 weeks. The DGLA of 314 mg/day is to be taken in terms of free fatty acid. The DGLA-containing oil was obtained by the method described in Japanese Patent No. 3354581. Specifically, to 200 g of dry cells of a filamentous fungus (Mortierella alpina), 500 mL of hexane was added, the mixture was stirred, and the filtration was repeated three times to extract oil, and then the hexane layer was evaporated to obtain DGLA-containing oil. The subjects took 4 capsules filled with this oil per day.

The placebo group: Olive oil (which does not contain the DGLA; refined olive oil manufactured by DSP Gokyo Food & Chemical Co., Ltd. (currently Sumitomo Pharm Food & Chemical Co., Ltd.)) was taken at 1,000 mg/day for 15 weeks.

### (3) Intake Duration of Diet for Testing

The intake duration of the diet for testing was 15 weeks from January 8th to April 23rd, 2022. Note that in Tokyo, where the testing was performed, the first day of Japanese cedar pollen dispersal was February 15th, which was 38 days after the start day of intake of the diet for testing (January 8th), and pollen dispersal continued until the end of the testing.

### (4) Measurement Items

Main Outcome Measures: The severity classification of allergic rhinitis symptoms and the Japanese Allergic Rhinitis Standard Quality of Life Questionnaire (JRQLQ)

Secondary Outcome Measures: Allergen-specific IgE concentrations in blood (house dust, mites, Japanese cedar, and hinoki cypress), histamine levels in blood, IL-6 levels in blood, and eosinophil count in nasal discharge.

Allergen-specific IgE concentrations in blood were measured by the fluorescence enzyme immunoassay (FEIA) method. Histamine levels in blood were measured by the enzyme immunoassay (EIA) method. IL-6 levels in blood were measured by the electrochemiluminescence immunoassay (ECLIA) method. The eosinophil count in nasal discharge was measured by the Wright's stain method.

### (5) Statistical Analysis

The mean ± SD of the change in score from the start of the testing was calculated for each group at each time point, and statistical analysis was conducted. For the analysis, mixed-effect model repeated measures analysis was used.

### 2. Results

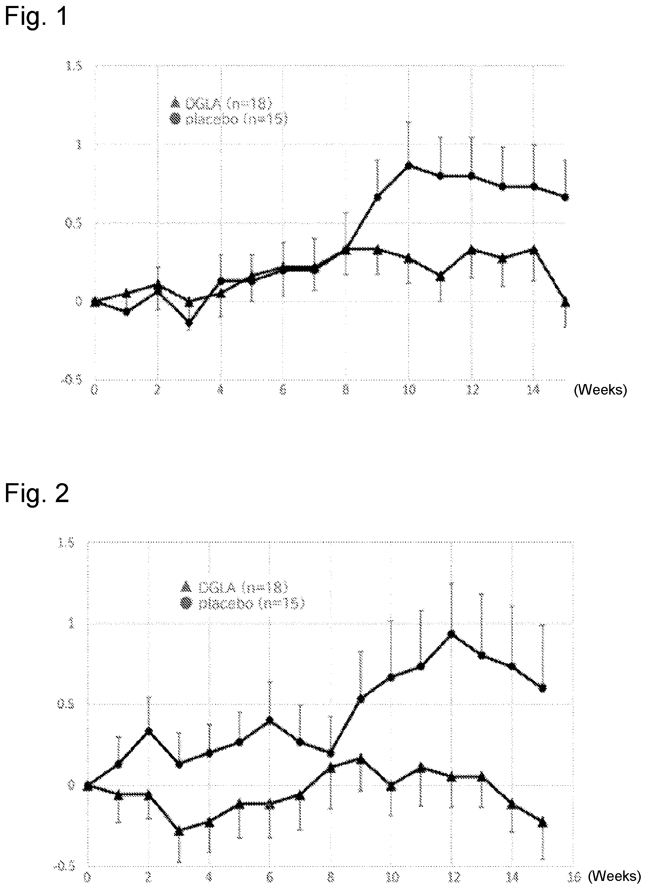

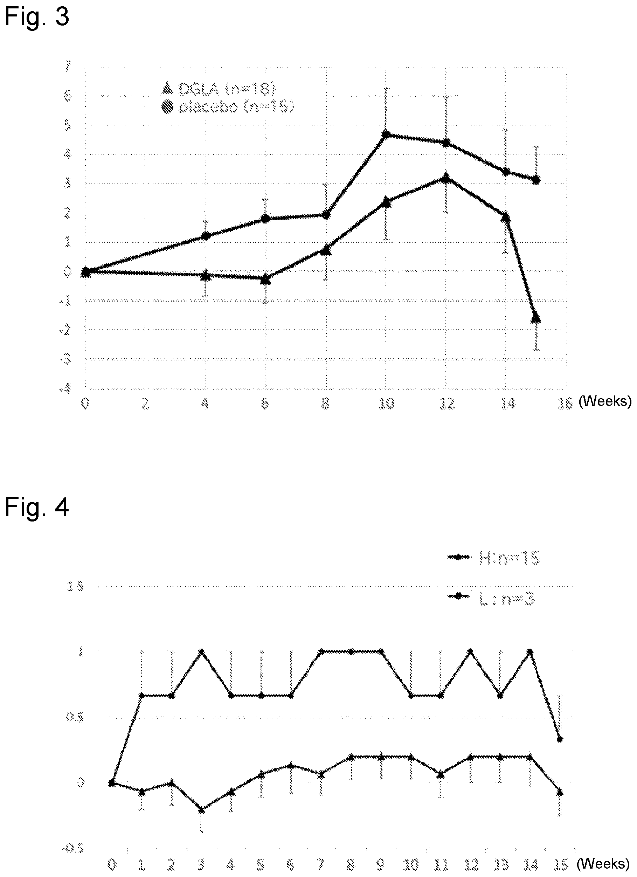

For the severity classification of allergic rhinitis symptoms, sneezing attack and nasal congestion were significantly reduced in the DGLA group (Figs. 1 and 2; p < 0.01 for sneezing attack, and p < 0.05 for nasal congestion). Also, for the JRQLQ, the nasal and eye symptom score (i.e., the sum of the scores of runny nose, sneezing, blocked nose, itchy nose, itchy eyes, and watery eyes) tended to be reduced in the DGLA group (Fig. 3; p < 0.1).

Further, stratification analysis was conducted on the DGLA group.

First, the DGLA group was divided into those who have a score of 1 or higher on each item about nose and eye (sneezing attack, nasal discharge, nasal congestion, itchy eyes, and tearing) in the severity classification at week 0 (at the time of the start of the testing) (Group H: those with mild symptoms or severer), and those who have a score of 0 (Group L: those with no symptom at all), and the results on the testing were compared.

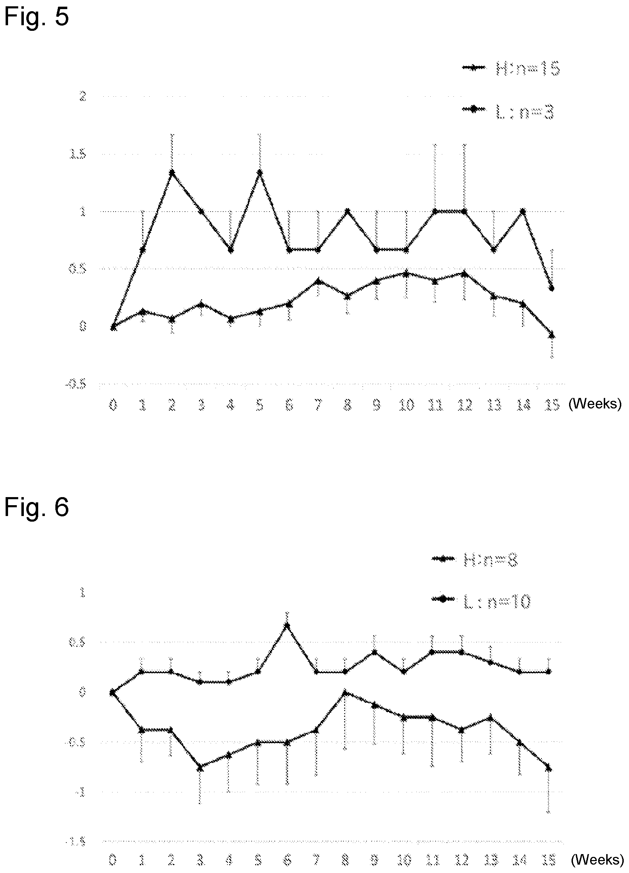

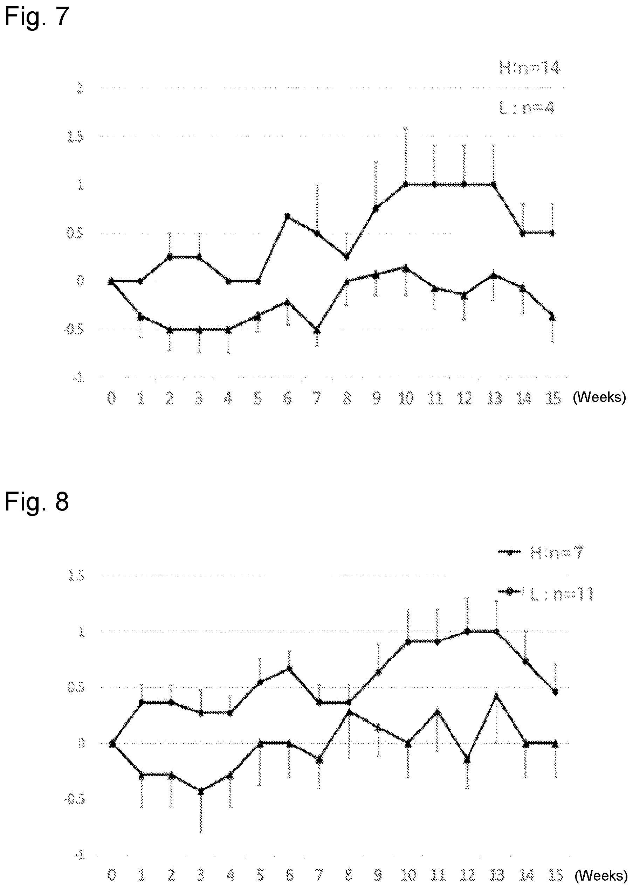

As a result, no significant difference between the groups was observed, but all the mean values for the items of Group H were lower than those of Group L (Figs. 4 to 8; P=0.712 for sneezing attack, P=0.258 for nasal discharge, P=0.249 for nasal congestion, and P=0.284 for tearing). Hence, it was confirmed that the DGLA has higher effects on the subjects who had experienced nasal or eye symptoms before pollen dispersal, in other words, on the subjects highly sensitive to pollen.

Also, the DGLA group was divided into those who have a score of 1 or higher on each item about nose and eye (runny nose, sneezing, blocked nose, itchy nose, itchy eyes, and watery eyes) in the JRQLQ at week 0 (at the time of the start of the testing) (Group H: those with mild symptoms or severer), and those who have a score of 0 (Group L: those with no symptom at all), and the results on the testing were compared.

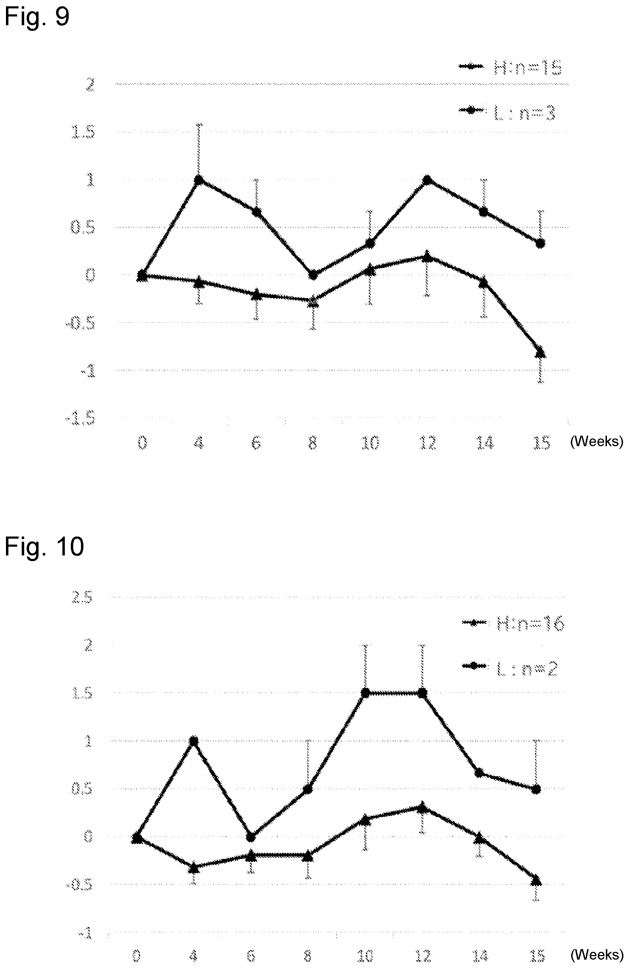

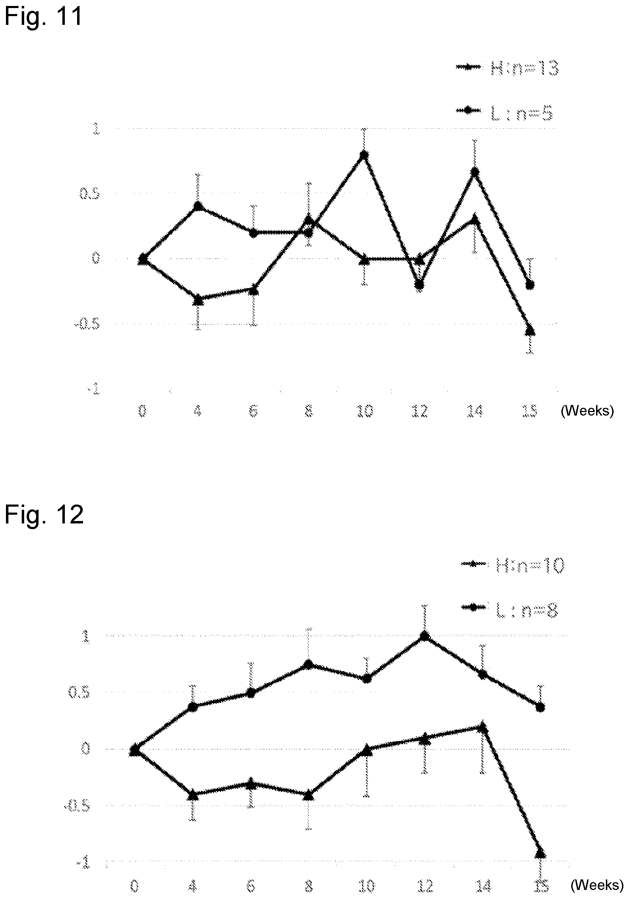

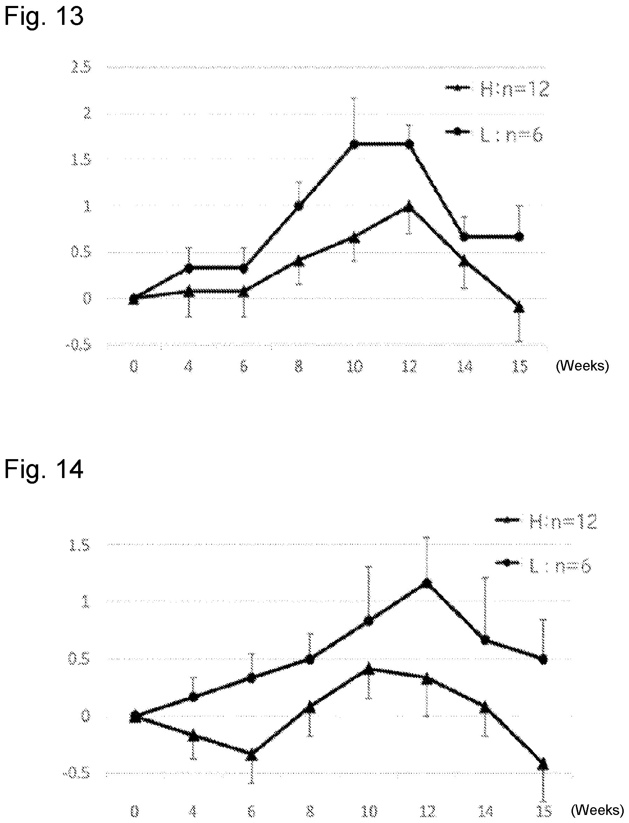

As a result, no significant difference between the groups was observed, but all the mean values for the items of Group H were lower than those of Group L (Figs. 9 to 14; P=0.582 for runny nose, P=0.345 for sneezing, P=0.126 for blocked nose, P=0.084 for itchy nose, P=0.362 for itchy eyes, and P=0.59 for watery eyes). Hence, it was confirmed that the DGLA has higher effects on the subjects who had experienced nasal or eye symptoms before pollen dispersal, in other words, on the subjects highly sensitive to pollen.

In addition, the mean value of the hinoki cypress pollen-specific IgE concentrations in serum in the DGLA group at week 0 (at the time of the start of the testing) was 1.98 AU/mL in the subjects who had the itchy nose score of 0 (Group L) in the JRQLQ at week 0. In contrast, the mean value for the subjects who had the score of 1 or higher (Group H) was 0.73 AU/mL, which was significantly lower than the value for the subjects who had the score of 0 (Group L).

### INDUSTRIAL APPLICABILITY

The present invention provides a pharmaceutical composition, a food composition, a food additive, and the like for alleviating or preventing a symptom of allergic rhinitis or allergic conjunctivitis.

## Claims

1. A composition for alleviating or preventing a symptom of allergic rhinitis or allergic conjunctivitis, comprising dihomo-γ-linolenic acid (DGLA) as an active ingredient.

2. The composition according to claim 1, wherein the allergic rhinitis or allergic conjunctivitis is pollinosis.

3. The composition according to claim 2, wherein the composition is used for oral administration to a subject having the symptom of the allergic rhinitis or allergic conjunctivitis at an early stage of a pollen dispersal period.

4. The composition according to claim 3, wherein at least one score selected from sneezing attack, nasal discharge, nasal congestion, itchy eyes, and tearing in a severity classification of allergic rhinitis symptoms for the subject is 1 or higher at the early stage of the pollen dispersal period.

5. The composition according to claim 3, wherein at least one score selected from runny nose, sneezing, blocked nose, itchy nose, itchy eyes, and watery eyes in the Japanese Allergic Rhinitis Standard Quality of Life Questionnaire for the subject is 1 or higher at the early stage of the pollen dispersal period.

6. The composition according to claim 3, wherein a hinoki cypress pollen-specific IgE concentration in serum of the subject is 0.73 UA/mL or less.

7. The composition according to any one of claims 1 to 6, wherein the symptom is at least one selected from sneezing attack, nasal discharge, nasal congestion, itchy nose, itchy eyes, and tearing.

8. The composition according to claim 7, wherein the symptom is at least one selected from sneezing attack, nasal discharge, itchy eyes, and tearing.

9. The composition according to any one of claims 1 to 8, wherein the DGLA of 4 mg/kg body weight/day or more is used for oral administration to the subject.

10. The composition according to any one of claims 1 to 9, wherein the DGLA is in any form of a glyceride, a phospholipid, a glycolipid, an alkyl ester, a salt, or a free fatty acid.

11. The composition according to claim 10, wherein the glyceride is a triglyceride, a diglyceride, or a monoglyceride.

12. The composition according to claim 10, wherein the alkyl ester is an ester with a lower alcohol having 1 to 4 carbon atoms.

13. The composition according to claim 10, wherein the phospholipid is a diacylglycerophospholipid or a lysoacylglycerophospholipid.
